Europäisches Patentamt

European Patent Office

Office européen des brevets

(11) Numéro de publication : **0 340 105 B1**

(12) **FASCICULE DE BREVET EUROPEEN**

(45) Date de publication du fascicule du brevet :
04.03.92 Bulletin 92/10

(51) Int. Cl.⁵ : **A61K 9/52, A61K 31/55**

(21) Numéro de dépôt : **89401180.8**

(22) Date de dépôt : **25.04.89**

(54) **Microbilles de diltiazem, leur procédé de fabrication et compositions pharmaceutiques à libération prolongée les contenant.**

(30) Priorité : **27.04.88 FR 8805629**

(43) Date de publication de la demande :
**02.11.89 Bulletin 89/44**

(45) Mention de la délivrance du brevet :
**04.03.92 Bulletin 92/10**

(84) Etats contractants désignés :
**AT BE CH DE ES FR GB GR IT LI LU NL SE**

(56) Documents cités :
**EP-A- 0 099 109
EP-A- 0 149 920
EP-A- 0 207 041
EP-A- 0 216 743
GB-A- 2 091 203**

(73) Titulaire : **SANOFI
40, Avenue George V
F-75008 Paris (FR)**

(72) Inventeur : **Desmolin, Henri
27 rue Fontaine d'Arlac
F-33700 Merignac (FR)**

(74) Mandataire : **Le Guen, Gérard et al
CABINET LAVOIX 2, place d'Estienne d'Orves
F-75441 Paris Cédex 09 (FR)**

## Description

La présente invention concerne une forme galénique à libération prolongée du diltiazem, antagoniste calcique utilisé dans le traitement de l'angor et de l'hypertension artérielle, depuis plusieurs années.

On sait que la demi-vie de ce principe actif est courte, de l'ordre de 4 heures, et le médicament est actuellement administré sous forme de comprimés trois à quatre fois par jour, ce qui est d'autant plus astreignant pour le patient que son traitement doit être maintenu pendant des périodes prolongées. En outre, avec l'administration de cette forme pharmaceutique, la concentration plasmatique varie fortement entre deux prises, de telle sorte que l'efficacité thérapeutique n'est pas continue, et qu'il existe, au moment des pics de concentration, un risque accru d'effets secondaires.

Il est par conséquent, souhaitable d'avoir une forme pharmaceutique qui permettre une libération prolongée, sensiblement constante de ce principe actif, de telle sorte qu'il soit possible d'administrer le médicament seulement une ou deux fois par jour.

On connait différents moyens de réalisation de formes pharmaceutiques à libération prolongée, administrables par voie orale, que ce soit sous forme de comprimés, ou sous forme de microgranules, telles que celles décrites dans EP-A-0 076 428, EP-A-0 061 217 et EP-A-0 216 743.

De tels moyens doivent être adaptés, pour chaque médicament, puisque la vitesse et la durée de la libération du principe actif, ainsi que sa concentration plasmatique dépendent de ses caractéristiques physico-chimiques, dont sa solubilité et sa stabilité dans le tractus gastro-intestinal, et de ses paramètres pharmacocinétiques.

Une telle forme est particulièrement difficile à obtenir pour les composés à demi-vie courte, inférieur à 4-5 heures, comme le diltiazem, puisque la durée de la libération in vivo à partir de la forme pharmaceutique ne dépasse pas le temps de séjour de la forme dans le milieu gastro-intestinal, c'est-à-dire 8 à 10 heures au maximum, et cela si la libération commence dès l'arrivée dans l'estomac.

En outre, pendant cette période la libération devra être sensiblement constante bien que le pH du milieu environnant évolue de 1,5 dans l'estomac à 6,9 dans le jejunum.

EP-A-0 207 041 décrit un comprimé renfermant des microgranules qui comprennent un film d'enrobage protecteur extérieur, non collant, fortement lubrifié de manière à permettre une séparation rapide des microgranules lorsque le comprimé est mis en contact avec un milieu aqueux.

Le film d'enrobage peut certes contenir du talc mais la fonction de ce talc est d'être un lubrifiant.

GB-A-2 091 203 décrit un procédé de préparation de microcapsules non collantes, qui consiste à précipiter autour des noyaux actifs insolubles, un polymère filmogène en solution par addition d'un non-solvant dans le milieu; les microgranules obtenus porteurs d'une membrane donnent une cinétique de libération de l'ordre de quelques minutes.

EP-A-0 149 920 décrit une forme à libération prolongée de diltiazem, constituée de microgranules dont le noyau comporte, outre le principe actif, un mélange de polymère et d'acide organique; ce dernier est indispensable pour obtenir une libération prolongée.

L'un des objets de la présente invention est une composition pharmaceutique de diltiazem à libération prolongée, qui selon le type de traitement, ne nécessite qu'une ou deux administrations journalières par voie orale. Elle est constituée d'une gélule ou capsule de gélatine, d'amidon ou d'autre polymère rapidement biodégradable dans le milieu gastrique, contenant une multiplicité de microbilles, formées d'un noyau contenant le principe actif entouré d'une membrane microporeuse, cette membrane étant composée d'un polymère filmogène et d'une charge représentant 35 à 75% en poids de la membrane, la quantité de principe actif et l'épaisseur de la membrane étant tels que la vitesse de libération du diltziem, mesurée par la technique de l'United States Pharmacopea dans des milieux de dissolution artificiels de pH allant de 1,5 à 7, soit constante pendant au moins 6 heures, après un temps de latence inférieur à 1 heure.

Le noyau peut être constitué de sphérules, obtenues par extrusion-sphéronisation d'une masse plastique, à base d'un polyol comme le mannitol ou d'un polymère comme un polyglycol, et d'un sel hydrosoluble de diltiazem, mais on préfère un noyau constitué d'un grain support constitué d'un excipient neutre, ce grain support étant enrobé du principe actif.

Le grain support peut être constitué d'un sucre, avec un hydrocolloïde, tel que la gomme arabique, la gélatine ou l'amidon ou d'un polymère biocompatible comme les celluloses micro-cristallines, les alkylcelluloses, les carboxyméthylcelluloses, ainsi que de leurs mélanges ou d'autres excipients bien connus de l'homme de l'art. On y ajoute éventuellement une charge minérale comme le talc. Le grain support peut être préparé de façon classique en turbine, ou par extrusion/sphéronisation de polymères ou par granulation d'une masse fondue passant par un orifice vibrant avant refroidissement.

Le principe actif est ensuite fixé sur le grain support, en une seule fois ou en couches successives, en turbine, ou en lit fluidisé soit par pulvérisation d'une solution ou suspension du sel de diltiazem et d'un polymère liant avec évaporation du solvant, soit par pulvérisation d'une solution alcoolique du liant sur le grain suivie de la pulvérisation du sel de diltiazem en poudre sur la couche visqueuse déposée; on préfère la seconde solution car les grains ainsi obtenus forment moins d'agrégats et sont plus réguliers.

De préférence il ne doit plus rester une quantité notable de principe actif dans les microbilles lorsqu'elles quittent in vivo la zone duodénale de résorption, ce qui correspond in vitro à la libération d'au moins 60% du principe actif du noyau en 8 heures, et la concentration du noyau en principe actif est calculée en conséquence.

En général, pour des raisons techniques, il est préférable que la concentration du noyau en principe actif soit de 30 à 50% en poids, mais notamment pour les compositions pharmaceutiques selon l'invention à plus fort dosage, on peut avoir jusqu'à 85% de principe actif dans le noyau.

Il est souhaitable d'avoir des microbilles de diamètre compris entre 0,4 et 1,4 mm, de telle sorte que le nombre de celles-ci, introduit dans une gélule de dimension acceptable pour que le patient l'avale sans difficulté, soit supérieur à 100 et de préférence compris entre 200 et 600; ainsi la dose du principe actif et sa cinétique de libération seront d'une reproductibilité acceptable d'une gélule à l'autre malgré l'hétérogénéité inévitable des microbilles due aux techniques de fabrication. Dans ces conditions les noyaux auront de préférence des diamètres compris entre 0,4 mm et 1,3 mm. Dans le cas où le principe actif est fixé par un liant sur le grain support, on utilise de 5 à 20% en poids de liant par rapport au poids du sel de diltiazem. Les liants utilisables sont des liants hydrosolubles bien connus de la technique, compatibles avec le sel de diltiazem, tels que des méthylcelluloses, des polyacrylates hydrosolubles et des polyvinylpyrrolidones. On utilise de préférence une polyvinylpyrrolidone de masse moléculaire voisine de 50 000, commercialisée par GAF (RFA) sous la marque Plasdone.

Comme sel de diltiazem, on peut utiliser en général le sel classique, à savoir le chlorhydrate, mais on pourrait utiliser d'autres sels plus ou moins hydrosolubles, soit d'acides minéraux, comme le sulfate, soit d'acides organiques, comme le fumarate, l'oxalate, le succinate et autres équivalents; la quantité de principe actif dans le noyau et l'épaisseur de la membrane seront définis en fonction de la solubilité aqueuse du sel.

Un aspect fondamental de l'invention réside dans le choix des caractéristiques de la membrane microporeuse qui entoure le noyau.

En effet, on a constaté que pour obtenir une forme pharmaceutique à libération prolongée, ayant une cinétique in vivo convenable, il fallait que la vitesse de diffusion in vitro du sel de diltiazem à partir des microbilles, dans les milieux de dissolution artificiels classiques de pH allant de 1,5 à 7 soit sensiblement constante pendant au moins 6 heures et de préférence jusqu'à 8 heures, et que la période nécessaire à l'obtention du régime régulier de diffusion, ou temps de latence, soit inférieur à 1 heure.

Dans ces conditions, des gélules contenant 120 mg de principe actif inclus dans des microbilles de l'invention, administrées 2 fois par jour chez l'homme, donnent, à l'état d'équilibre, une concentration plasmatique toujours supérieure à 80 ng/ml, avec un pic voisin de 130 ng/ml atteint en 6 heures, alors qu'une composition pharmaceutique classique administrée 2 fois par jour aux mêmes doses, donne rapidement un pic de concentration s'élevant jusqu'à 180 ng/ml avec, après 6 heures, une décroissance rapide jusqu'à 50 ng/ml.

Parmi les polymères biocompatibles, non hydrosolubles, stables in vivo pouvant constituer la membrane microporeuse, on peut citer les polyacrylates et polyméthacrylates du type Eudragit, les alkylcelluloses dont les méthylcelluloses du type Tylose (Hoechst) et les éthylcelluloses, telles que celles commercialisées par Hercules et les laques d'origine naturelle telles que les gommes shellac. Les propriétés de ces dernières sont peu reproductibles d'un lot à l'autre et on préfère l'éthylcellulose, de qualité constante et inerte chimiquement dont la viscosité, mesurée selon la méthode de US National Formulary, est comprise entre 10 et 50 mPa.s

Les polymères doivent être associés à un agent plastifiant pour que la membrane ne soit pas cassante.

Parmi les agents plastifiants, qui peuvent être notamment des esters phtaliques, des polyéthyléneglycols, l'huile de ricin, le glycérol, on préfère l'huile de ricin.

La présence d'une charge, en quantité suffisante, est fondamentale; elle diminue le temps de gonflement de la membrane in vivo, donc le temps de latence, sans augmenter simultanément exagérément la vitesse de diffusion du principe actif; une vitesse faible, nécessaire pour une forme à libération prolongée peut être obtenue en augmentant l'épaisseur d'une membrane de composition classique, mais le temps de latence s'accroit simultanément.

On sait que dans le cas du diltiazem, il est fondamental d'avoir un temps de latence court, pour éviter les pertes de biodisponibilité et une libération du principe actif pendant une période trop courte avant la sortie des microbilles du tractus gastro-intestinal. Pour diminuer ce temps de latence on ne peut cependant trop diminuer l'épaisseur de la membrane, ce qui rendrait sa réalisation difficilement reproductible quant à ses propriétés de diffusion; l'hétérogénéité qui en résulterait pour les billes ainsi obtenues dans un même lot ou dans des lots successifs rendrait pratiquement impossible la réalisation de compositions pharmaceutiques dont le taux de libération du principe actif soit défini et reproductible.

Parmi les charges finement divisées utilisables, insolubles dans le solvant d'application de la membrane, on peut citer le talc, la silice, des silicates métalliques tels que les silicates d'Al et Mg, le kaolin, le lactose et le saccharose pulvérisés, des oxydes métalliques comme l'oxyde de titane, ou leurs mélanges. On préfère les charges neutres, comme le talc.

La membrane microporeuse peut être appliquée en pulvérisant une dispersion alcoolique ou hydroalcoolique du polymère filmogène, du plastifiant et de la charge en turbine ou en lit d'air fluidisé.

Dans le cas d'une membrane à bas d'éthylcellulose, son épaisseur sera comprise entre 15 micromètres et 60 micromètres.

La quantité de la composition à déposer pour former une membrane d'épaisseur convenable, est déterminée par des essais préalables au cours desquels on mesure la vitesse de diffusion du diltiazem in vitro dans les conditions standard de l'United States Pharmacopea (USO 21. Chap. 711, page 1243; appareil n° 1) en utilisant des milieux de dissolution artificiels, de différents pH, allant de 1,5 à 7 et par exemple 1,5 et 4,5 et 7, à partir des microbilles comportant le noyau choisi et des membranes d'épaisseurs différentes, déposées en couches successives. On choisit alors l'épaisseur de la membrane de telle sorte qu'une vitesse de diffusion sensiblement constante s'établisse en moins d'une heure et on déduit de la valeur mesurée du flux de diffusion des microbilles ayant cette épaisseur de membrane quelles doivent être les caractéristiques dimensionnelles du noyau pour assurer la libération in vitro par heure de 10% environ de la quantité du principe actif présent.

On a effectivement constaté, que ce taux de libération in vitro, donnait un vivo des concentrations plasmatiques satisfaisantes pour l'usage thérapeutique envisagé.

Les microbilles de l'invention sont stables; les cinétiques de dissolution in vitro ne sont pas modifiées au cours du stockage, comme on le constate fréquemment avec ce type de forme pharmaceutique.

Les compositions pharmaceutiques selon l'invention peuvent se présenter sous forme de gélules de composition et de taille classiques, contenant de 100 à 600 microbilles de l'invention et de préférence de 200 à 350, de telle sorte qu'une unité de dose contienne de 90 mg à 350 mg de diltiazem. On introduira dans chaque gélule le nombre de microbilles nécessaire ; on peut utiliser les mêmes microbilles quelle que soit la dose unitaire à réaliser, mais on préfère notamment, pour les doses extrêmes, adopter la composition et la taille de la microbille de telle sorte que le nombre de microbilles par gélule soit compris entre 200 et 350.

On peut introduire ces microbilles dans d'autres formes pharmaceutiques : cachets, comprimés secables ou non, suppositoires ou suspensions liquides ou gélifiées.

Dans ce qui suit, on décrit des modes de réalisation particuliers donnés à titre d'exemples, des microbilles, de leur procédé de préparation et des nouvelles compositions pharmaceutiques à libération prolongée de l'invention. Les courbes de dissolution dans des milieux artificiels de différents pH, préparés comme décrit dans l'U.S.P., ont été tracés avec un appareil dans lequel on introduit l'équivalent en microbilles de la dose de principe actif par litre de milieu. Le chlorhydrate de diltiazem libéré en fonction du temps est dosé par spectrophotomètrie.

EXEMPLE :

Pour préparer environ 10 kg de grains support, on introduit dans une turbine environ 2 kg de "semence" obtenue par tamisage de saccharose cristallisé sur tamis d'ouverture 0,500 mm et dépoussièrée sur tamis d'ouverture 0,200 mm.

On pulvérise en plusieurs fois une solution hydroalcoolique constituée de saccharose (6 parties) et polyvidone K30 (polyvinylpyrrolidone de masse moléculaire moyenne 40 000) (une partie) dans un mélange eau-alcool éthylique (50/50-V/V), soit environ 5 litres pour 2,5 kg de mélange saccharose/polyvidone.

Entre chaque mouillage, on poudre avec un mélange de talc (une partie), d'amidon de mais (une partie) et de saccharose (2 parties) jusqu'à obtenir des microsphères de diamètre environ 0,5 à 0,6 mm. Après séchage en étuve à 40°C, la fraction de diamètre compris entre 0,600 mm et 0,300 mm est isolée; elle sert de grais supports dans ce qui suit.

On introduit environ 1,5 kg de grains support dans une turbine et on double environ le poids en mouillant avec une solution alcoolique de polyvidone à 10% (p/v) et en poudrant entre chaque addition avec du chlorhydrate de diltiazem préalablement tamisé sur grille de 0,5 mm, jusqu'à obtention d'environ 2,5 kg de noyaux. On sèche à l'éuve à 40°C.

Le litre en principe actif est de l'ordre de 40% en poids. On élimine les petits noyaux sur grille de 0,6 mm et on remet les noyaux dans une turbine, munie d'un dispositif de pulvérisation. On pulvérise alors sur les noyaux, en saupoudrant de talc entre chaque addition, une solution alcoolique à environ 12% en poids du mélange éthylcellulose (5 parties) et huile de ricin (1 partie); l'éthylcellulose a une viscosité de 18 à 24 mPa.s.

Quand la masse des billes a augmenté de 10% on effectue un premier prélèvement A, puis on poursuit le dépôt de la membrane jusqu'à une augmentation de 30% en poids, avant d'effectuer un prélèvement B, et de 55% pour donner les microbilles C; ces trois lots sont séchés à l'étuve à 40°C. On détermine la cinétique de dissolution in vitro à pH 1,5, pour chacun d'eux, d'où l'on déduit le débit horaire en régime constant (pourcentage de principe actif libéré par la microbille en 1 heure), ainsi que le temps de latence.

Les résultats obtenus figurent dans le tableau ci-dessous :

| Prise d'essai correspondant à 120 mg de principe actif | : | Temps de latence | : | Débit horaire |
|---|---|---|---|---|
| Prélèvement A | : | 0 | | 25% |
| Prélèvement B | : | 2 heures | | 13,75% |
| Prélèvement C | : | 4 heures | | 9% |

Dans ce cas, on constate que les microbilles B et C ne répondaient pas aux caractéristiques de l'invention (temps de latence trop élevé) et que la membrane devait être plus épaisse que celle des microbilles A (dissolution trop rapide) et l'on a modifié les conditions opératoires en conséquence, pour obtenir des microbilles selon l'invention.

On a ainsi préparé des microbilles permettant d'avoir une dose de 120 mg de principe actif dans une gélule n° 1 avec 250 microbilles par gélule; ces microbilles comportent 48% en poids de principe actif, des grains supports représentant 31% du poids de la microbille tandis que la membrane représente 17,5% en poids de la microbille.

On a aussi préparé des microbilles pour avoir une dose de 300 mg de principe actif dans une gélule n° 0, à partir de grains supports représentant 11% du poids final de la microbille, qui contenait 75% en poids de principe actif; la membrane représentant 11% en poids de la microbille.

In vitro, ces microbilles ne présentent pratiquement pas de temps de latence, le débit de libération est régulier et la quantité de diltiazem libérée au bout de 4 heures est environ 40% de la quantité totale tandis qu'elle est d'environ 60% au bout de 6 heures.

Ces caractéristiques ne sont pas modifiées après au moins 18 mois de conservation à la température ambiante et après 3 mois à 40°C ou à 55°C.

**Revendications**

1. Microbilles de diltiazem qui sont constituées d'un noyau comprenant le principe actif entouré d'une membrane microporeuse constituée d'un polymère filmogène insoluble en milieu aqueux, d'un plastifiant représentant 10 à 30% en poids dudit polymère filmogène, et d'une charge représentant 35 à 75% en poids de la membrane, la quantité de principe actif et l'épaisseur de la membrane étant tels que la vitesse de libération du diltaziem, mesurée par la technique de l'United States Pharmacopea dans des milieux de dissolution artificiels de pH allant de 1,5 à 7, soit constante pendant au moins 6 heures, après un temps de latence inférieur à 1 heure.

2. Microbilles selon la revendication 1, caractérisées en ce que le diltiazem est sous forme de sel hydrosoluble.

3. Microbilles selon la revendication 2, caractérisées en ce que le diltiazem est sous forme de chlorhydrate.

4. Microbilles selon l'une quelconque des revendications 1 à 3, caractérisées en ce qu'elles ont un diamètre compris entre 0,4 et 1,4 mm.

5. Microbilles selon l'une quelconque des revendications 1 à 4, caractérisée en ce que le noyau est constitué d'un grain support inerte enrobé d'une couche de principe actif fixé dans un liant.

6. Microbilles selon la revendication 5, caractérisées en ce que le liant est de la polyvinyl-pyrrolidone.

7. Microbilles selon l'une quelconque des revendications 1 à 6, caractérisées en ce que le polymère filmogène est de l'éthylcellulose.

8. Microbilles selon la revendication 7, caractérisées en ce que l'éthylcellulose a une viscosité comprise entre 10 et 50 mPa.s.

9. Microbilles selon l'une quelconque des revendications 1 à 8, caractérisées en ce que le plastifiant est de l'huile de ricin.

10. Microbilles selon l'une quelconque des revendications 1 à 9, caractérisées en ce que la charge est du talc, du kaolin, de la silice, un silicate métallique ou un oxyde métallique.

11. Microbilles selon la revendication 10, caractérisées en ce qu'elles ont un diamètre compris entre 0,4 et 1,4 mm et que l'épaisseur de la membrane est compris entre 15 micromètres et 60 micromètres.

12. Procédé de préparation des microbilles selon l'une quelconque des revendications 1 à 11, caractérisé en ce que la membrane est appliquée sur le noyau par pulvérisation d'une dispersion de ses constituants dans un solvant.

13. Procédé selon la revendication 12, caractérisé en ce que la membrane est déposée en couches successives.

14. Composition pharmaceutique à libération prolongée de diltiazem, caractérisée en ce qu'elle est constituée de gélules contenant des microbilles selon l'une quelconque des revendications 1 à 11.

15. Composition pharmaceutique selon la revendication 14, caractérisée en ce que la gélule contient de 100 à 600 microbilles.

16. Composition pharmaceutique selon l'une des revendications 14 et 15, caractérisée en ce que la gélule contient de 90 à 350 mg de diltiazem.

**Revendications pour les Etats contractants suivants : ES GR**

1. Procédé de préparations de microbilles de diltiazem qui sont constituées d'un noyau comprenant le principe actif entouré d'une membrane microporeuse constituée d'un polymère filmogène insoluble en milieu aqueux, d'un plastifiant représentant 10 à 30% en poids dudit polymère filmogène, et d'une charge représentant 35 à 75% en poids de la membrane, la quantité de principe actif et l'épaisseur de la membrane étant tels que la vitesse de libération du diltiaziem, mesurée par la technique de l'United States Pharmacopea dans des milieux de dissolution artificiels de pH allant de 1,5 à 7, soit constante pendant au moins 6 heures, après un temps de latence inférieur à 1 heure, procédé consistant à appliquer la membrane sur le noyau par pulvérisation d'une dispersion de ses constituants dans un solvant.

2. Procédé selon la revendication 1, caractérisé en ce que la membrane est déposée en couches successives.

3. Procédé selon la revendication 2, caractérisé en ce que le diltiazem est sous forme de sel hydrosoluble.

4. Procédé selon la revendication 3, caractérisé en ce que le diltiazem est sous forme de chlorhydrate.

5. Procédé selon la revendication 1, caractérisé en ce que l'on prépare des microbilles ayant un diamètre compris entre 0,4 et 1,4 mm.

6. Procédé selon la revendication 1 ou la revendication 2, caractérisé en ce que l'on prépare des microbilles ayant un noyau est constitué d'un grain support inerte enrobé d'une couche de principe

7. Procédé selon la revnedication 6, caractérisé en ce que l'on utilise de la polyvinylpyrrolidone comme liant.

8. Procédé selon l'une quelconque des revendications 1 à 7, caractérisé en ce que l'on utilise de l'éthylcellulose comme polymère filmogène.

9. Procédé selon la revendication 8, caractérisé en ce que l'on utilise de l'éthylcellulose ayant une viscosité comprise entre 10 et 50 mPa.s.

10. Procédé selon l'une quelconque des revendications 1 à 9, caractérisé en ce que l'on utilise de l'huile de ricin comme plastifiant.

11. Procédé selon l'une quelconque des revendications 1 à 10, caractérisé en ce que l'on utilise comme charge est du talc, du kaolin, de la silice, un silicate métallique ou un oxyde métallique.

12. Procédé selon la revendication 11, caractérisé en ce que l'on prépare des microbilles dont le diamètre est compris entre 0,4 et 1,4 mm et l'épaisseur de la membrane est comprise entre 15 micromètres et 60 micromètres.

13. Procédé de préparation d'une composition pharmaceutique à libération prolongée de diltiazem, caractérisé en ce que l'on prépare des gélules contenant des microbilles préparées selon l'une quelconque des revendications précédentes.

14. Procédé de préparation d'une composition pharmaceutique selon la revendication 13, caractérisé en ce que l'on prépare une gélule qui contient de 100 à 600 microbilles.

15. Procédé de préparation d'une composition pharmaceutique selon l'une des revendications 13 et 14, caractérisé en ce que l'on prépare une gélule qui contient de 90 à 350 mg de diltiazem.

## Patentansprüche

1. Diltiazem-Mikrokugeln, die aus einem Kern bestehen, der den mit einer mikroporösen Membran umhüllten Wirkstoff enthält, wobei die Membran aus einem in wäßrigem Medium unlöslichen filmbildenten Polymer, einem Weichmacher, der 10 bis 30 Gew.-% des filmbildenden Polymers darstellt, und einem Füllstoff, der 35 bis 75 Gew.-% der Membran darstellt, besteht, und wobei die Wirkstoffmenge und die Dicke der Membran so sind, daß die nach dem Verfahren gemäß dem US-Arzneibuch gemessene Freisetzungsgeschwindigkeit des Diltiazems in künstlichen Lösungsmitteln mit einem pH-Wert von 1,5 bis 7 nach einer Latenzzeit unter einer Stunde über mindestens 6 Stunden konstant ist.

2. Mikrokugeln nach Anspruch 1, dadurch gekennzeichnet, daß das Diltiazem in Form eines wasserlöslichen Salzes vorliegt.

3. Mikrokugeln nach Anspruch 2, dadurch gekennzeichnet, daß das Diltiazem in Form des Hydrochlorids vorliegt.

4. Mikrokugeln nach einem der Ansprüche 1 bis 3, dadurch gekennzeichent, daß sie einen Durchmesser zwischen 0,4 und 1,4 mm aufweisen.

5. Mikrokugeln nach einem der Ansprüche 1 bis 4, dadurch gekennzeichnet, daß der Kern aus einem inerten Trägerkorn besteht, das mit einer Schicht von in einem Bindemittel gebundenem Wirkstoff umhüllt ist.

6. Mikrokugeln nach Anspruch 5, dadurch gekennzeichnet, daß das Bindemittel Polyvinylpyrrolidon ist.

7. Mikrokugeln nach einem der Ansprüche 1 bis 6, dadurch gekennzeichnet, daß das filmbildende Polymer Ethylcellulose ist.

8. Mikrokugeln nach Anspruch 7, dadurch gekennzeichnet, daß die Ethylcellulose eine Viskosität zwischen 10 und 50 mPa.s besitzt.

9. Mikrokugeln nach einem der Ansprüche 1 bis 8, dadurch gekennzeichnet, daß der Weichmacher Ricinusöl ist.

10. Mikrokugeln nach einem der Ansprüche 1 bis 9, dadurch gekennzeichnet, daß der Füllstoff Talk, Kaolin, Kieselsäure, ein Metallsilicat oder ein Metalloxid ist.

11. Mikrokugeln nach Anspruch 10, dadurch gekennzeichnet, daß sie einen Durchmesser zwischen 0,4 und 1,4 mm aufweisen und die Dicke der Membran zwischen 15 und 60 µm liegt.

12. Verfahren zur Herstellung der Mikrokugeln nach einem der Ansprüche 1 bis 11, dadurch gekennzeichnet, daß die Membran durch Aufsprühen einer Dispersion ihrer Bestandteile in einem Lösungsmittel auf den Kern aufgebracht wird.

13. Verfahren nach Anspruch 12, dadurch gekennzeichnet, daß die Membran in aufeinanderfolgenden Schichten aufgebracht wird.

14. Pharmazeutische Zusammensetzung mit verlängerter Freisetzung von Diltiazem, dadurch gekennzeichnet, daß sie aus Gelatinekapseln besteht, die Mikrokugeln nach einem der Ansprüche 1 bis 11 enthalten.

15. Pharmazeutische Zusammensetzung nach Anspruch 14, dadurch gekennzeichnet, daß die Kapseln 100 bis 600 Mikrokugeln enthalten.

16. Pharmazeutische Zusammensetzung nach einem der Ansprüche 14 und 15, dadurch gekennzeichnet, daß die Kapseln 90 bis 350 mg Diltiazem ertthalten.

## Patentansprüche für folgende Vertragsstaaten : ES GR

1. Verfahren zur Herstellung von Diltiazem-Mikrokugelng die aus einem Kern besteheng der den mit einer mikroporösen Membran umhüllten Wirkstoff enthält, wobei die Membran aus einem in wäßrigem Medium unlöslichen filmbildenden Polymer, einem Weichmacher, der 10 bis 30 Gew.-% des filmbildenden Polymers darstellt, und einem Füllstoff, der 35 bis 75 Gew.-% der Membran darstellt, besteht, und wobei die Wirkstoffmenge und die Dicke der Membran so sind, daß die nach dem Verfahren gemäß dem US-Arzneibuch gemessene Freisetzungsgeschwindigkeit von Diltiazem in künstlichen Lösungsmitteln mit einem pH-Wert von 1,5 bis 7 nach einer Latenzzeit unter einer Stunde über mindestens 6 Stunden konstant ist, das darin besteht, die Membran durch Aufsprühen einer Dispersion ihrer Bestandteile in einem Lösungsmittel auf den Kern aufzubringen.

2. Verfahren nach Anspruch 1, dadurch gekennzeichnet, daß die Membran in aufeinanderfolgenden Schichten aufgebracht wird.

3. Verfahren nach Anspruch 2, dadurch gekennzeichnet, daß das Diltiazem in Form eines wasserlöslichen Salzes vorliegt.

4. Verfahren nach Anspruch 3, dadurch gekennzeichnet, daß das Diltiazem in Form des Hydrochlorids vorliegt.

5. Verfahren nach Anspruch 1, dadurch gekennzeichnet, daß man Mikrokugeln mit einem Durchmesser zwischen 0,4 und 1,4 mm herstellt.

6. Verfahren nach Anspruch 1 oder 2, dadurch gekennzeichnet, daß man Mikrokugeln mit einem Kern herstellt, der aus einem inerten Trägerkorn besteht, das mit einer Schicht Wirkstoff umhüllt ist.

7. Verfahren nach Anspruch 6, dadurch gekennzeichnet, daß man als Bindemittel Polyvinylpyrrolidon verwendet.

8. Verfahren nach einem der Ansprüche 1 bis 7, dadurch gekennzeichnet, daß man als filmbildendes Polymer Ethylcellulose verwendet.

9. Verfahren nach Anspruch 8, dadurch gekennzeichnet, daß man Ethylcellulose mit einer Viskosität zwischen 10 und 50 mPa.s verwendet.

10. Verfahren nach einem der Ansprüche 1 bis 9, dadurch gekennzeichnet, daß man als Weichmacher Ricinusöl verwendet.

11. Verfahren nach einem der Ansprüche 1 bis 10, dadurch gekennzeichnet, daß man als Füllstoff Talk, Kaolin, Kieselsäure, ein Metallsilicat oder ein Metalloxid verwendet.

12. Verfahren nach Anspruch 11, dadurch gekennzeichnet, daß man Mikrokugeln herstellt, deren Durchmesser zwischen 0,4 und 1,4 mm liegt und wobei die Dicke der Membran zwischen 15 $\mu$m und 60 $\mu$m liegt.

13. Verfahren zur Herstellung einer pharmazeutischen Zusammensetzung mit verlängerter Freisetzung von Diltiazem, dadurch gekennzeichnet, daß man Gelatinekapseln herstellt, die nach einem der vorhergehenden Ansprüche hergestellte Mikrokugeln enthalten.

14. Verfahren zur Herstellung einer pharamzeutischen Zusammensetzung nach Anspruch 13, dadurch gekennzeichnet, daß man Kapseln herstellt, die 100 bis 600 Mikrokugeln enthalten.

15. Verfahren zur Herstellung einer pharmazeutischen Zusammensetzung nach einem der Ansprüche 13 und 14, dadurch gekennzeichnet, daß man Kapseln herstellt, die 90 bis 350 mg Diltiazem enthalten.

## Claims

1. Diltiazem microbeads which consist of a core containing the active ingredient surrounded by a microporous membrane consisting of a film-forming polymer insoluble in aqueous medium, a plasticizer representing 10 to 30% by weight of the film-forming polymer, and a filler representing 35 to 75% by weight of the membrane, the quantity of active ingredient and the thickness of the membrane being such that the rate of release of the diltiazem, measured by the technique of the United States Pharmacopea in artificial dissolution media ranging from pH 1.5 to 7, remains constant for at least 6 hours, after a latent period of less than one hour.

2. Microbeads according to claim 1, characterised in that the diltiazem is in the form of a water-soluble salt.

3. Microbeads according to claim 2, characterised in that the diltiazem is in the form of the hydrochloride.

4. Microbeads according to any one of claims 1 to 3, characterised in that they have a diameter between 0.4 and 1.4 mm.

5. Microbeads according to any one of claims 1 to 4, characterised in that the core consists of an inert grain substrate coated with a layer of active ingredient combined with a binder.

6. Microbeads according to claim 5, characterised in that the binder is polyvinyl pyrrolidone.

7. Microbeads according to any one of claims 1 to 6, characterised in that the film-forming polymer is ethylcellulose.

8. Microbeads according to claim 7, characterised in that the ethylcellulose has a viscosity of between 10 and 50 mPa.s.

9. Microbeads according to any one of claims 1 to 8, characterised in that the plasticizing agent is castor oil.

10. Microbeads according to any one of claims 1 to 9, characterised in that the filler is selected from talc, kaolin, silica, a metal silicate or a metal oxide.

11. Microbeads according to claim 10, characterised in that they have a diameter from 0.4 to 1.4 mm and the thickness of the membrane is from 15 microns to 60 microns.

12. A process for preparing the microbeads according to any one of claims 1 to 11, characterised in that the membrane is applied to the core by spraying a dispersion of its components in a solvent.

13. A process according to claim 12, characterised in that the membrane is deposited in successive layers.

14. A sustained-release pharmaceutical composition of diltiazem, characterised in that it consists of capsulescontaining microbeads according to any one of claims 1 to 11.

15. A pharmaceutical composition according to claim 14, characterised in that the capsule contains from 100 to 600 microbeads.

16. A pharmaceutical composition according to one of claims 14 and 15, characterised in that the capsule contains from 90 to 350 mg of diltiazem.

**Claims for the following Contracting States : ES GR**

1. A process for preparing microbeads of diltiazem which consist of a core containing the active ingredient surrounded by a microporous membrane consisting of a film-forming polymer insoluble in aqueous medium, a plasticizier representing 10 to 30% by weight of the film-forming polymer, and a filler representing 35 to 75% by weight of the membrane, the quantity of active ingredient and the thickness of the membrane being such that the rate of release of the diltiazem, measured by the technique of the United States Pharmacopea in artificial dissolution media ranging from pH 1.5 to 7, remains constant for at least 6 hours, after a latent period of less than one hour, said process consisting in applying the membrane to the core by spraying a dispersion of its components in a solvent.

2. A process according to claim 1, characterised in that the membrane is deposited in successive layers.

3. A process according to claim 2, characterised in that the diltiazem is in the form of a water-soluble salt.

4. A process according to claim 3, characterised in that the diltiazem is in the form of the hydrochloride.

5. A process according to claim 1, characterised in that microbeads are prepared which have a diameter of between 0.4 and 1.4 mm.

6. A process according to claim 1 or 2, characterised in that microbeads are prepared having a core which consists of an inert grain substrate coated with a layer of active ingredient.

7. A process according to claim 6, characterised in that polyvinylpyrrolidone is used as binder.

8. A process according to any one of claims 1 to 7, characterised in that ethylcellulose is used as the film-forming polymer.

9. A process according to claim 8, characterised in that ethylcellulose having a viscosity of between 10 and 50 mPa.s is used.

10. A process according to any one of claims 1 to 9, characterised in that castor oil is used as plasticiser.

11. A process according to any one of claims 1 to 10, characterised in that talc, kaolin, silica, a metal silicate or a metal oxide is used as the filler.

12. A process according to claim 11, characterised in that microbeads are prepared having a diameter of between 0.4 and 1.4 mm and with a membrane between 15 and 60 microns thick.

13. A process for preparing a sustained-release pharmaceutical composition of diltiazem, characterised in that gelatin capsules are prepared containing microbeads prepared according to any one of the preceding claims.

14. A process for preparing a pharmaceutical composition according to claim 13, characterised in that a gelatin capsule is prepared containing 100 to 600 microbeads.

15. A process for preparing a pharmaceutical composition according to one of claims 13 and 14, characterised in that a capsule is prepared containing 90 to 350 mg of diltiazem.